# FASCICULE DE BREVET EUROPEEN

(11) **EP 2 893 906 B1**
(45) Date de publication et mention de la délivrance du brevet: **07.07.2021**
(21) Numéro de dépôt: 15150358.8
(22) Date de dépôt: 07.01.2015
(51) Int. Cl.: A61F 2/38

(54) **Prothèse postero stabilisée du genou**
Rückwärtig stabilisierte Knieprothese
Posterior-stabilised knee prosthesis

(30) Priorité: 14.01.2014 FR 1450256
(43) Date de publication de la demande: 15.07.2015
(73) Titulaire: Evolutis, 42720 Briennon (FR)
(72) Inventeur: Massin, Philippe, 75116 Paris (FR); Durand, Jean-Marc, 18000 Bourges (FR); Peguet, Jean-Michel, 42840 Montagny (FR); Meire, Philippe, 86100 Chatellerault (FR); Huguet, Dominique, 44300 Nantes (FR); Jardin, Christophe, 76310 Sainte Adresse (FR); Viale, Patrick, Gilles, 18000 Bourges (FR); Cotte, Jean-Luc, 89240 Beauvoir (FR); Ehkirch, François-Paul, 78290 Croissy Sur Seine (FR)
(74) Mandataire: Cabinet Laurent & Charras

(56) Documents cités:
- EP-A1- 0 970 667
- EP-A1- 2 438 889
- FR-A1- 2 769 494
- FR-A1- 2 809 302
- FR-A1- 2 913 591
- US-A1- 2009 326 666
- US-A1- 2012 143 342

## Description

L'invention se rattache au secteur technique des implants orthopédiques et concerne, plus particulièrement, une prothèse du genou.

L'invention trouve une application avantageuse dans le cas d'une prothèse tricompartimentale du genou notamment du type à glissement. Ce type de prothèse comprend, de manière parfaitement connue pour un homme du métier, un implant fémoral et un implant tibial, lesquels implants ne sont pas accouplés par des moyens de liaison mécanique contrairement aux prothèses totales du genou dites à charnières. L'implant fémoral est constitué par une trochlée prolongée, de part et d'autre d'une échancrure, par deux patins condyliens coopérant en appui avec capacité de flexion et de rotation, avec des compartiments généralement de profils complémentaires, que présente un plateau tibial le plus souvent réalisé en polyéthylène. Le plateau tibial est rendu solidaire de l'embase tibiale soit de manière fixe soit avec capacité de mobilité. L'implant fémoral et l'implant tibial, notamment l'embase tibiale qui reçoit le plateau, présentent tout type d'agencement pour être fixés dans la partie articulaire anatomique de l'os correspondant après y avoir effectué des coupes fémorales et tibiales nécessaires.

Avec ce type de prothèse, le problème de la postérostabilité, notamment en flexion, a été posé et différentes solutions ont été proposées pour le résoudre.

On a ainsi proposé d'équiper l'implant fémoral et l'implant tibial de moyens de guidage et/ou de butée complémentaires lors du mouvement de flexion. Par exemple, ces moyens peuvent être constitués par une butée transversale disposée au niveau de l'échancrure intercondylienne et apte à coopérer avec une butée formée en débordement du plateau tibial par exemple. Ce type de solution ressort par exemple de l'enseignement du brevet FR 2 809 302, du brevet EP 0 970 667 et des demandes de brevet US 2012 / 143 342 A1, EP 2 438 889 A1 et US 2009 / 326 666 A1.

Toutefois, ces solutions ne permettent pas de résoudre, de manière satisfaisante, le problème posé par l'hyperflexion de l'articulation du genou comme différentes études l'ont démontré. On rappelle que l'hyperflexion passive du genou permet par exemple de s'asseoir sur les talons ou bien de forcer manuellement la flexion en essayant de faire toucher le talon aux fesses. Or, une prothèse tricompartimentale du genou ne le permet pas, et peut limiter également la flexion active qui a lieu entre environ 60° et 120° ce qui interdit de nombreuses activités.

Sur un plan anatomique, la flexion active est limitée par le conflit entre le rebord postérieur tibial et la corticale fémorale tandis que l'hyper flexion passive suppose un soulèvement du condyle médial en raison du conflit postérieur et une subluxation postérieure du condyle latéral.

Le retour vers l'avant du condyle latéral en extension est assuré par le ligament croisé antérieur tandis que la stabilité du condyle médial est assurée par la tension du ligament croisé postérieur. Or, une prothèse tricompartimentale est généralement utilisée lorsque l'appareil ligamentaire est défectueux, autrement dit, une telle prothèse n'est plus apte à jouer la fonction précédemment citée.

Il résulte, par conséquent, de l'état antérieur de la technique, que dans le cas d'une prothèse tricompartimentale du genou, des risques importants de luxation peuvent apparaitre en flexion active et qu'il est très difficile, voire impossible, d'obtenir une flexion passive.

L'invention s'est fixée pour but de remédier à ces inconvénients de manière simple, sûre, efficace et rationnelle.

Le problème que se propose de résoudre l'invention est d'obtenir une postéro stabilisation de la prothèse au fur et à mesure de la flexion depuis une position dite « d'hyper extension » lors de la flexion pour la marche et lors de la flexion active jusqu'à sensiblement 120° avec la possibilité d'obtenir une flexion passive au-delà de 120° jusqu'à 140° environ.

Plus particulièrement le problème posé est résolu par les caractéristiques relevant de la deuxième partie de la revendication 1.

Pour résoudre le problème posé d'obtenir un contact linéaire de l'élément profilé au fur et à mesure de la flexion et de permettre une décoaptation c'est-à-dire un soulèvement de la partie postérieure des patins condyliens, l'élément profilé présente latéralement à l'intérieur de l'échancrure un rebord d'appui constituant le profil en came. Les sections latérale et transversale de l'élément génèrent sensiblement une partie de sphère.

Le problème posé est également résolu en ce que la face d'appui du plateau tibial en contact avec l'élément est concave latéralement et transversalement, les concavités latérale et transversale étant complémentaires des sections latérale et transversale dudit élément.

Pour résoudre le problème posé de toujours assurer un contact de l'élément profilé en came et faisant office de troisième condyle, y compris en cas d'extension passive, le plot est raccordé avec le bord postérieur du plateau par une empreinte en creux profilée d'une manière complémentaire à la section transversale profilée de l'élément reliant les deux patins condyliens.

Selon une autre caractéristique, les patins condyliens présentent sur une partie de leur longueur, un rayon de courbure constant identique à celui des empreintes du plateau tibial formées de part et d'autre du plot.

Compte tenu des caractéristiques à la base de l'invention et du problème posé à résoudre, le profil en came de l'élément est en contact avec le profil latéral du plot à partir de sensiblement 50° de flexion de l'élément fémoral jusqu'à sensiblement 100°.

Le profil en came de l'élément est en contact avec le profil latéral du plot à partir de sensiblement 50° de flexion de l'élément fémoral jusqu'à sensiblement 100° où il commence à échapper ledit profil latéral et à coopèrer tel un troisième condyle dans l'empreinte en creux, la dite empreint ayant une profondeur significativement inférieure à celle qui permettrait le maintien du contact des deux condyles avec les deux empreintes du plateau tibial.

Selon une autre caractéristique, l'échancrure délimite entre les deux patins condyliens, une cage creuse pour l'engagement et le passage du plot du plateau tibial au fur et à mesure de la flexion.

De manière avantageuse, le plateau est monté avec capacité de rotation sur l'embase tibiale.

L'invention est exposée ci-après plus en détail à l'aide des figures des dessins annexés dans lesquels :

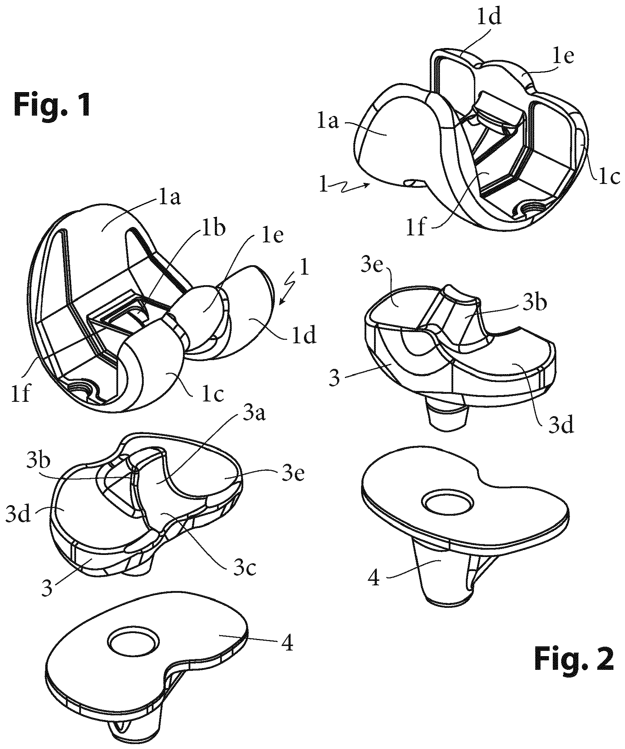

- les figures 1 et 2 sont des vues en perspective avant mise en place de l'élément fémoral, du plateau tibial et de l'embase tibiale ;
- les figures 3 et 4 sont des vues en perspective correspondant respectivement aux figures 1 et 2 après mise en place de l'implant fémoral, du plateau tibial et de l'embase tibiale ;
- les figures 5 et 6 sont des vues schématiques de côté de l'implant tibial et du plateau tibial en position d'hyper extension respectivement à -10° et 0° ;
- les figures 7 et 8 sont de vues correspondant aux figures 5 à 6 et considérées au niveau de l'élément profilé et du plot de centrage et de guidage du plateau tibial ;
- les figures 9, 10, 11, 12 et les figures 13, 14, 15, 16 sont des vues correspondant respectivement aux figures 5, 6, 7 et 8 montrant la position de flexion pour la marche à 0° de flexion (figures 9 et 13), 20° de flexion (figures 10, 14), 50° de flexion (figures 11,15) et 60° de flexion, (figures 12, 16) ;
- les figures 17, 18, 19, 20 et 21, 22, 23, 24 sont des vues correspondant respectivement aux figures 9, 10, 11, 12 et 13, 14, 15, 16 en position de flexion active à 60° de flexion (figures 17 et 21), 80° de flexion (figures 18 et 22), 100° de flexion (figures 19 et 23) et 120° de flexion (figures 20 et 24) ;
- les figures 25, 26, 27 et 28, 29, 30 sont des vues correspondant respectivement aux figures 17, 18, 19, 20 et 21, 22, 23, 24 en position de flexion passive à 120° de flexion (figures 25, 28), 130° de flexion (figures 26, 29) et 140° de flexion (figures 27 et 30).

La prothèse de genou comprend, de manière connue, un implant fémoral (1) et un implant tibial (2). L'implant fémoral (1) est constitué par une trochlée (1a) prolongée de part et d'autre d'une échancrure (1b) par deux patins condyliens (1c) et (1d). Les patins condyliens (1c) et (1d) coopèrent avec capacité de flexion et de rotation avec les compartiments interne et externe d'un plateau tibial (3) généralement en polyéthylène. Comme il sera indiqué dans la suite de la description, le plateau tibial (3) est monté sur une embase métallique (4).

Les deux patins condyliens (1c) et (1d) sont réunis transversalement à leurs extrémités postérieures au niveau de l'extrémité libre de l'échancrure (1b) par un élément de profilé (1e). Cet élément (1c) est conformé en sections tant latérale que transversale, pour faire respectivement office de came (1e1) et de troisième condyle (1e2). Le profil en came (1e1) est constitué par un rebord d'appui orienté en direction de l'échancrure inter condylienne (1b). De manière importante, les sections latérale et transversale de l'élément profilé (1e) génèrent sensiblement une partie de sphère.

Cet élément profilé (3) coopère facialement avec une surface d'appui profilée (3a) que présente un plot (3b) formé en débordement du plateau tibial (3). Comme il sera développé dans la suite de la description, cet élément profilé (1e) qui fait office de came et de troisième condyle permet une flexion postéro stabilisée au fur et à mesure de la flexion depuis une position d'extension et au-delà de 120° avec décoaptation d'une partie des patins condyliens dans une position de flexion passive de l'ordre de 130° à 140°.

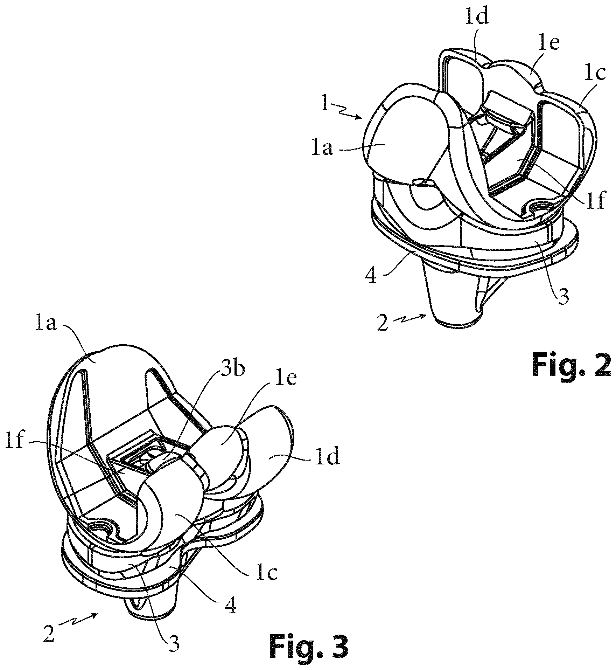

La face d'appui (3a) du plot (3b) du plateau du tibia (3), est concave latéralement et transversalement de manière complémentaire à la section latérale et transversale de l'élément (1e) correspondant au mouvement de flexion combiné avec le mouvement de rotation de l'élément fémoral lors de ladite flexion. Autrement dit, ces dispositions permettent une posterio stabilisation parfaite au fur et à mesure de la flexion respectant parfaitement l'anatomie. Comme le montre notamment la figure 3, le plot (3b), notamment sa face d'appui (3a), est raccordé avec le bord postérieur du plateau (3) par une empreinte en creux (3c) profilée de manière complémentaire à la section transversale de l'élément (1e). Ces caractéristiques permettent de loger et de guider l'extrémité postérieure de l'élément profilé (1e) notamment en position de flexion au-delà de 20°.

A noter également que l'échancrure (1b) délimite entre les deux patins condyliens (1c) et (1d) une cage creuse (1f) pour l'engagement et le passage du plot (3b) au fur et à mesure de la flexion. Cette cage (1f) participe également à la stabilité latérale de l'élément fémoral par rapport à l'implant tibial.

Les patins condyliens (1c) et (1d) présentent sur une partie de leur longueur un rayon de courbure constant identique à celui des empreintes congruentes (3d) et (3e) du plateau tibial, formées de part et d'autre du plot (3b).

Le profil (1e1) de l'élément (1e) est en contact avec le profil latéral du plot (3b) à partir de sensiblement 50° de flexion de l'implant fémoral jusqu'à sensiblement 100°. Au-delà, jusqu'à sensiblement 140°, c'est le troisième condyle (1e2) qui coopère en appui avec le profil latéral du plot (3b).

Au-delà 120°, et plus particulièrement aux environs de 130°, le profil transversal du troisième condyle (1e2) est déterminé pour créer une décoaptation de l'extrémité postérieure des patins condyliens correspondant à une flexion passive. Lors de cette décoaptation, la congruence est totale entre le profil latéral du plot et le profil de la came.

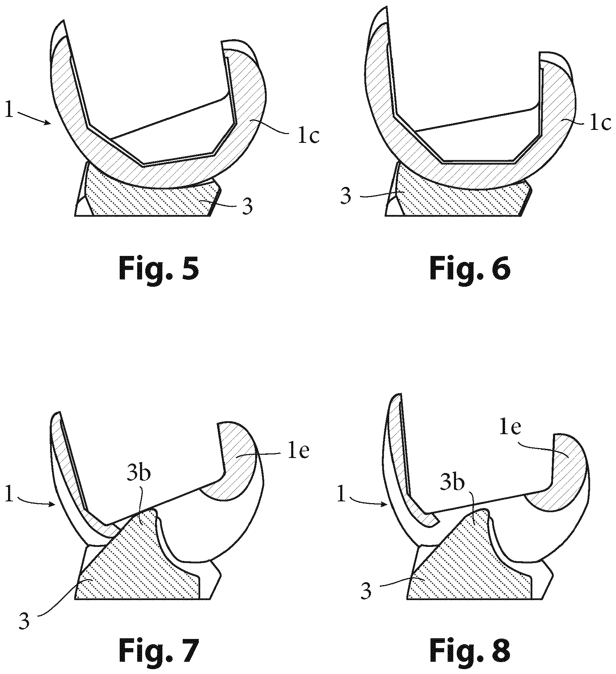

On renvoie à la cinématique de la prothèse du genou selon les caractéristiques de l'invention en se référant aux figures 5 à 30 des dessins.

Aux figures 5 à 8, l'implant fémoral est positionné en hyper extension par rapport à l'implant tibial, par exemple, à -10° (figures 5 et 7) ou à 0° (figures 6 et 8). Les patins condyliens sont parfaitement en appui sur la totalité de la surface des empreintes correspondantes du plateau tibial. L'élément profilé (7c) n'est pas en contact avec le plot (3b) et le plateau tibial.

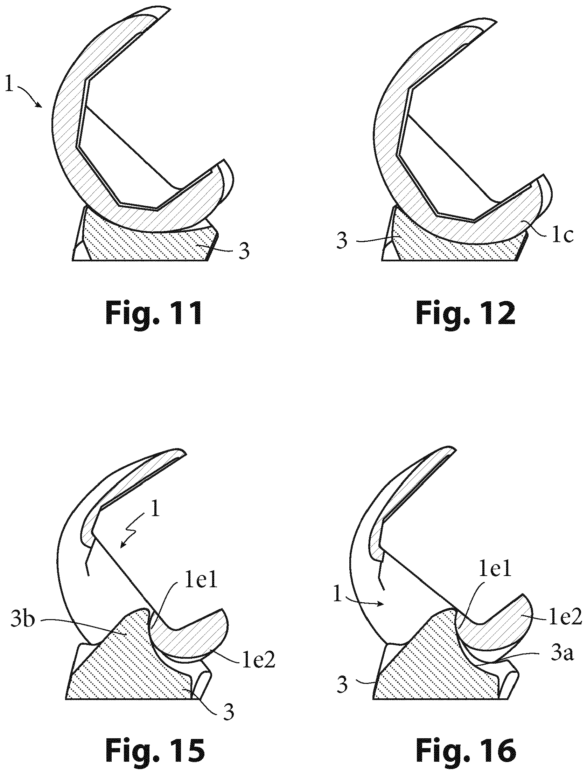

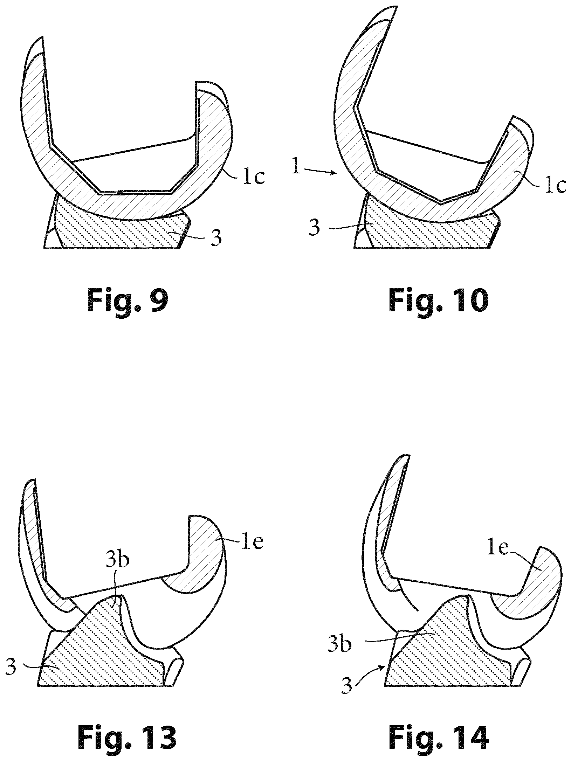

Les figures 9 à 16 montrent l'élément fémoral en position de flexion pour la marche, à 0° de flexion (figures 9 et 13), à 20° de flexion (figures 10 et 14), à 47° de flexion (figures 11 et 15), à 60° de flexion (figures 12 et 16). Dans la position illustrée figures 9, 10 et 11, les patins condyliens présentent un rayon de courbure constant, identique à celui des empreintes du plateau tibial.

A partir de 50° environs, l'élément (le) par son profil en came (1e1) est en contact avec la face (3a) du plot (3b), les patins condyliens commençant à s'écarter du plateau tibial aux environs d'un angle de flexion de 60° (figures 12 et 16).

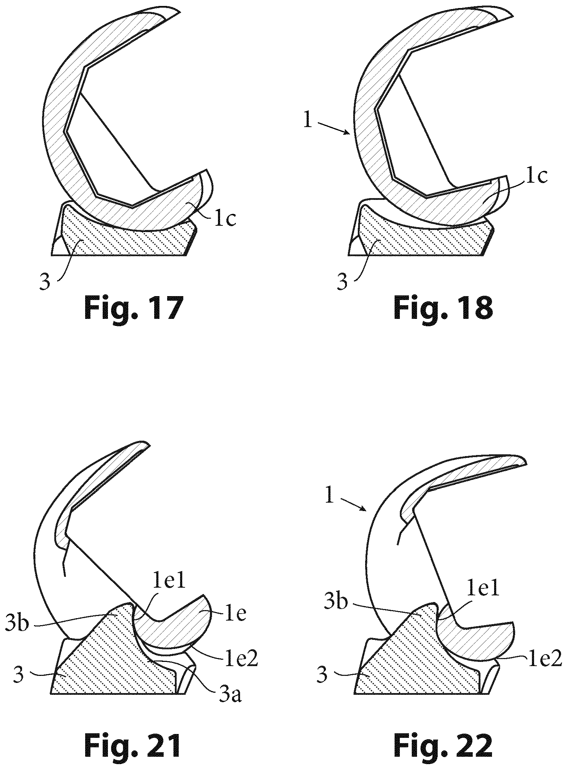

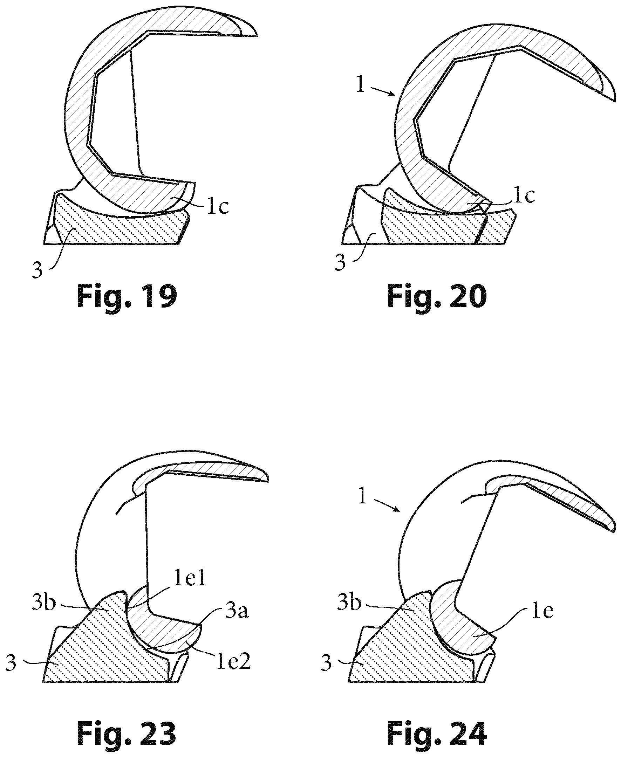

Les figures 17 à 24 montrent l'implant fémoral en position de flexion active, en position de 60° (figures 17 à 21), de 80° (figures 18 et 22), de 100° (figures 19 et 23) et de 120° (figures 20 et 24). Sur ce figures, on voit notamment le décollement progressif des patins condyliens par rapport au plateau tibial (figures 17, 18, 19 et 20) et le déplacement de l'élément (le) du profil en came (1e1) par rapport au plot du plateau tibial jusqu'à ce que le dit élément vienne totalement en contact avec ledit plot à 120° de flexion correspondant à la fonction de troisième condyle, en observant qu'à 100° de flexion les patins condyliens sont en contact limité avec la plateau tibial (figures 20 et 24).

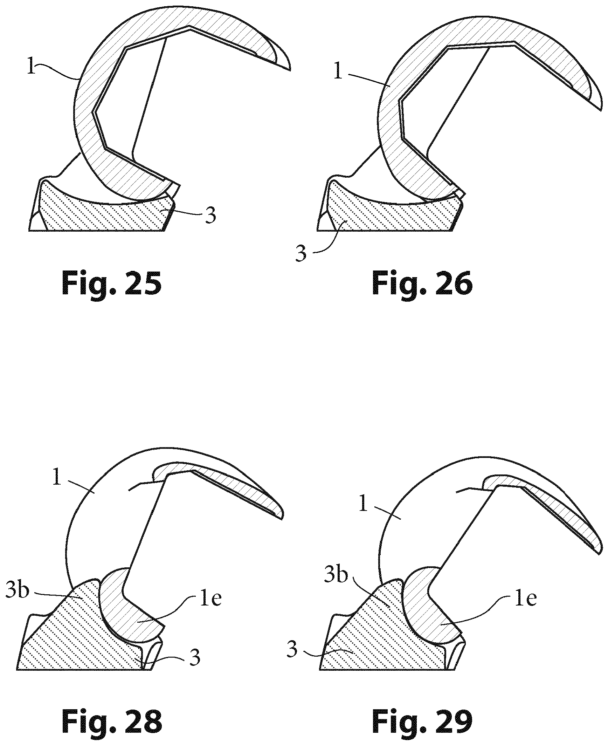

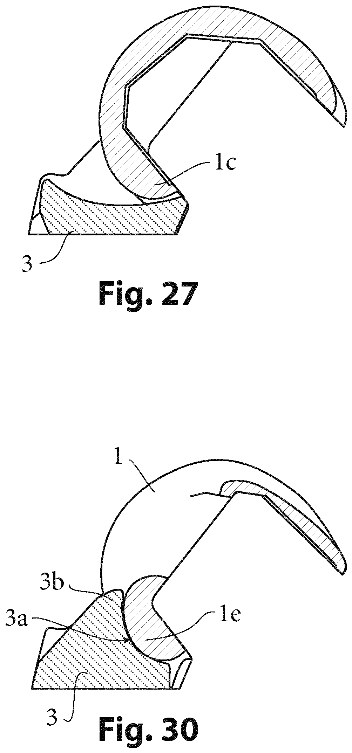

Les figures 25 à 30 montrent l'implant fémoral en position de flexion passive à 120° (figures 25 à 28), à 130° (figures 26 et 29) et à 140° (figures 27 et 30). Sur les figures 28, 29 et 30 on voit que le troisième condyle (1e2) demeure toujours en contact avec le plot (3b) tandis qu'à partir de 130° le profil transversal de troisième condyle est déterminé pour créer une décoaptation de l'extrémité postérieure des patins condyliens (figures 26 et 27).

Ces différentes caractéristiques permettent de respecter parfaitement l'anatomie du genou en permettant de résoudre de manière satisfaisante le problème posé par l'hyper flexion des prothèses du genou lorsque les différents ligaments ne sont plus opérationnels.

Avantageusement, le plateau tibial (3) est monté avec capacité de rotation sur l'embase tibiale (4), sans pour cela exclure une réalisation selon laquelle le plateau (3) est fixe.

## Revendications

1. Prothèse postéro stabilisée du genou comprenant un implant fémoral (1) et un implant tibial (2), l'implant fémoral (1) étant constitué par une trochlée (1a) prolongée de part et d'autre d'une échancrure (1b) par deux patins condyliens (1c) et (1d) coopérant avec capacité de flexion et de rotation avec des empreintes congruentes (3d) et (3e) d'un plateau tibial (3) que présente une embase constitutive de l'implant tibial (2), les deux patins condyliens (1c) et (1d) étant réunis transversalement à leur extrémité postérieure au niveau de l'extrémité libre de l'échancrure, par un élément profilé (le) en sections latérale et transversale pour faire office de came (1e1) et de troisième condyle (1e2) en coopérant respectivement avec des surfaces d'appui profilées (3a) et (3c) d'un plot (3b) formé en débordement du plateau tibial (3) pour permettre une flexion postéro stabilisée jusqu'à 120°, et au-delà de 120°, le soulèvement d'un des condyles (1c) ou (1d) afin qu'il n'entre plus en contact avec l'une des empreintes (3d) ou (3e) du plateau tibial (3), de sorte que lors du soulèvement la décoaptation est totale entre l'un des deux condyles et l'empreinte correspondante du plateau tibial, ledit profil en came (1e1) est en contact avec le profil latéral (3a) du plot (3b) à partir de sensiblement 50° de flexion de l'élément fémoral (1) jusqu'à sensiblement 100° où il commence à échapper audit profil latéral (3a) et à coopérer, tel un troisième condyle (1e2), avec la surface d'appui profilée (3c) constituant une empreinte en creux, dont la profondeur est significativement inférieure à celle qui permettrait le maintien du contact des deux condyles (1c) ou (1d) avec les deux empreintes (3d) ou (3e) du plateau tibial (3), **caractérisé en ce que** les surfaces d'appui (3a) et (3c) sont concaves latéralement et transversalement, et **en ce que** les concavités latérale et transversale sont complémentaires des sections latérale et transversale dudit élément (1e).

2. Prothèse selon la revendication 1 **caractérisée en ce que** l'élément profilé (1e) présente latéralement à l'intérieur de l'échancrure (1b) un rebord d'appui constituant le profil en came (1e1).

3. Prothèse selon la revendication 1 **caractérisée en ce que** les sections latérale et transversale de l'élément (1e) génèrent sensiblement une partie de sphère.

4. Prothèse selon la revendication 1 **caractérisée en ce que** le plot (3b) est raccordé avec le bord postérieur du plateau (3) par l'empreinte en creux (3c) profilée d'une manière complémentaire à la section transversale profilée de l'élément reliant les deux patins condyliens (1c) et (1d).

5. Prothèse selon la revendication 1 **caractérisée en ce que** les patins condyliens (1c) et (1d) présentent sur une partie de leur longueur, un rayon de courbure constant identique à celui des empreintes du plateau tibial formées de part et d'autre du plot (3b).

6. Prothèse selon la revendication 1 **caractérisée en ce que** l'échancrure délimite entre les deux patins condyliens (1c) et (1d), une cage creuse pour l'engagement et le passage du plot (3b) du plateau tibial (3) au fur et à mesure de la flexion.

7. Prothèse selon la revendication 1 **caractérisée en ce que** le plateau tibial (3) est monté avec capacité de rotation ou pas sur l'embase tibiale (4).

## Patentansprüche

1. Posterior- stabilisierte Knieprothese mit einem Femur- Implantat (1) und einem Tibia- Implantat (2), das Femur- Implantat (1) besteht dabei aus einer Trochlea (1a), beiderseits einer Vertiefung (1b), verlängert durch zwei Kondylenpolster (1c) und (Id) arbeitet, mit Flexions- und Rotationskapazität, mit kongruenten Abdrücken (3d) und (3e) eines Tibiaplateaus (3) zusammen, das eine wesentliche Basis des Tibia-Implantats (2) bildet, die beiden Kondylenpolster 10 (1c) und (1d) sind dabei in Querrichtung an ihrem posterioren Ende in Höhe des freien Endes der Vertiefung über ein Profilelement (1e) mit seitlichen und querlaufenden Einschnitten verbunden, um als Knorren (1e1) und dritter Kondylus (1e2) zu dienen, wobei sie jeweils mit den profilierten Auflageflächen (3a) und (3c) eines Stifts (3b) zusammenarbeiten, der aus dem Tibiaplateau (3) hervorragend geformt ist, um eine posterior- stabilisierte Flexion bis 120° zu ermöglichen und über 120° hinaus das Anheben eines der Kondylen (1c) oder (1d), so dass dieser keinen Kontakt mehr mit einem der Abdrücke (3d) oder (3e) des Tibiaplateaus (3) hat, so dass es beim Anheben zu einer vollständigen Dekoaptation zwischen einem der beiden Kondylen und dem entsprechenden Abdruck des Tibiaplateaus kommt, dieses nockenförmige Profil (1e1) steht in Kontakt mit dem seitlichen Profil (3a) des Stifts (3b), ab einer Flexion des Femurelementes (1) von im Wesentlichen 50° bis zu im Wesentlichen 100°, wo es beginnt aus diesem seitlichen Profil (3a) zu gleiten und, wie ein dritter Kondylus (1e2), mit der profilierten Auflagefläche (3c) zusammenzuarbeiten, die einen Hohlabdruck bildet, dessen Tiefe deutlich geringer ist als notwendig wäre, um den Kontakt der beiden Kondylen (1c) oder (1d) mit den beiden Abdrücken (3d) oder (3e) des Tibiaplateaus (3) aufrechtzuerhalten, **dadurch gekennzeichnet, dass** die Auflageflächen (3a) und (3c) seitlich und in Querrichtung konkav sind, und dass die seitlichen und querlaufenden Wölbungen komplementär zu den seitlichen und querlaufenden Einschnitten (1e) dieses Elementes sind.

2. Prothese nach Anspruch 1, **dadurch gekennzeichnet, dass** das Profilelement (1e) seitlich, innerhalb der Vertiefung (1b), einen Auflagerand aufweist, der das nockenförmige Profil bildet (1e1).

3. Prothese nach Anspruch 1, **dadurch gekennzeichnet, dass** die seitlichen und querlaufenden Einschnitte des Elementes (1e) im Wesentlichen einen Kugelteil bilden.

4. Prothese nach Anspruch 1, **dadurch gekennzeichnet, dass** der Stift (3b) mit dem posterioren Rand des Plateaus (3) über den profilierten Hohlabdruck (3c) in komplementärer Weise zum querlaufenden Profileinschnitt des die beiden Kondylenpolster (1c) und (1d) verbindenden Elementes, verbunden ist.

5. Prothese nach Anspruch 1, **dadurch gekennzeichnet, dass** die Kondylenpolster (1c) und (1d) auf einem Teil ihrer Länge einen konstanten Krümmungsradius aufweisen, der mit dem der Abdrücke des Tibiaplateaus, die beiderseits des Stifts (3b) gebildet sind, identisch ist.

6. Prothese nach Anspruch 1, **dadurch gekennzeichnet, dass** die Vertiefung zwischen den beiden Kondylenpolstern (1c) und (1d), einen hohlen Käfig bildet, zum Einsetzen und Durchführen des Stiftes (3b) des Tibiaplateaus (3) entsprechend der Flexion.

7. Prothese nach Anspruch 1, **dadurch gekennzeichnet, dass** das Tibiaplateau (3), mit Rotationskapazität oder ohne, auf der Tibiabasisplatte (4) montiert ist.

## Claims

1. A posterior-stabilized knee prosthesis comprising a femoral implant (1) and a tibial implant (2), the femoral implant (1) being constituted by a trochlea (1a) extended on either side of a notch (1b) by two condylar pads (1c) and (1d) cooperating with flexion and rotation capacity with congruent recesses (3d) and (3e) in a tibial tray (3) with which a base forming the tibial implant (2) is provided, the two condylar pads (1c) and (1d) being joined together transversely at their posterior ends at the free end of the notch by a shaped-section element (1e) that is shaped into a lateral section and into a transverse section, which sections act respectively as a cam (1e1) and as a third condyle (1e2) by cooperating with respective ones of shaped-section bearing surfaces (3a) and (3c) on a stud (3b) projecting from the tibial tray (3) to allow posteriorly-stabilized flexion up to 120°, and, beyond 120°, to allow one of the condyles (1c) or (1d) to be raised so that it no longer comes into contact with one of the recesses (3d) or (3e) in the tibial tray (3), such that, during such raising, de-coaptation is total between one of the two condyles and the corresponding recess in the tibial tray, and said cam shaped section (1e1) is in contact with the lateral shaped section (3a) of the stud (3b) from substantially 50° of flexion of the femoral element (1) to substantially 100°, whereupon it begins to escape from said lateral shaped section (3a) and to cooperate, in the manner of a third condyle (1e2), with the shaped-section bearing surface (3c) forming a recess, which has a depth significantly shallower than a depth that would enable the two condyles (1c) or (1d) to stay in contact with the two recesses (3d) or (3e) in the tibial tray (3), said posterior-stabilized knee prosthesis being **characterized in that** the bearing surfaces (3a) and (3c) are concave laterally and transversely, and **in that** the lateral and transverse concavities are complementary to the lateral and transverse sections of said element (1e).

2. The prosthesis according to claim 1, **characterized in that**, inside the notch (1b), the shaped-section element (1e) laterally has a bearing rim that constitutes the cam shaped section (1e1).

3. The prosthesis according to claim 1, **characterized in that** the lateral and transverse sections of the element (1e) substantially generate a portion of a sphere.

4. The prosthesis according to claim 1, **characterized in that** the stud (3b) is connected to the posterior edge of the tray (3) via the recess (3c) that is of shaped section complementary to the transverse shaped section of the element interconnecting the two condylar pads (1c) and (1d).

5. The prosthesis according to claim 1, **characterized in that**, over a portion of their length, the condylar pads (1c) and (1d) have a constant radius of curvature identical to that of the recesses in the tibial tray that are formed on either side of the stud (3b).

6. The prosthesis according to claim 1, **characterized in that**, between the two condylar pads (1c) and (1d), the notch delimits a well enabling the stud (3b) of the tibial tray (3) to be engaged into it and to pass through it as the flexion is taking place.

7. The prosthesis according to claim 1, **characterized in that** the tibial tray (3) is mounted with or without rotation capacity on the tibial base (4).
